(19) Europäisches Patentamt
European Patent Office
Office européen des brevets

(11) **EP 4 324 926 A1**

(12) **EUROPEAN PATENT APPLICATION**
published in accordance with Art. 153(4) EPC

(43) Date of publication:
**21.02.2024 Bulletin 2024/08**

(21) Application number: **22788395.6**

(22) Date of filing: **12.04.2022**

(51) International Patent Classification (IPC):
**C12N 15/87** (2006.01)       **C12M 1/42** (2006.01)
**C12M 3/06** (2006.01)

(52) Cooperative Patent Classification (CPC):
**C12M 1/42; C12M 3/06; C12N 15/87**

(86) International application number:
**PCT/KR2022/005272**

(87) International publication number:
**WO 2022/220539 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.04.2021 KR 20210047258**

(71) Applicant: **MxT Biotech**
**Seoul 04785 (KR)**

(72) Inventors:
• **CHUNG, A-Ram**
  **Seoul 04425 (KR)**
• **HUR, Jeong-Soo**
  **Seoul 06289 (KR)**

(74) Representative: **Meissner Bolte Partnerschaft mbB**
**Patentanwälte Rechtsanwälte**
**Postfach 86 06 24**
**81633 München (DE)**

(54) **INTRACELLULAR DELIVERY PLATFORM**

(57)     The present invention relates to an intracellular delivery platform including: a first channel through which a fluid comprising cells and delivery materials flows, and a second channel and a third channel which are connected to the first channel at an angle and through which the fluid comprising cells and delivery materials flows, wherein the fluid forms at least one of a collision region and a vortex region of the fluid in at least one of the first channel, the second channel, and the third channel. According to the present invention, material can be effectively delivered into cells by forming a vortex in channels.

FIG. 1

## Description

### TECHNICAL FIELD

The present disclosure relates to a platform which delivers intracellular materials, and more particularly, to a platform capable of delivering materials within cells by the formation of a vortex.

### BACKGROUND ART

Intracellular material transfer is one of the most basic experiments in cell engineering, and materials are usually delivered using carriers or by creating nanopores in cell membranes/nuclear membranes. Virus- or Lipofectamine-based carrier techniques can deliver materials with high efficiency when optimized, but have problems such as safety, slow delivery speed, labor/cost-intensive carrier preparation process, and low reproducibility.

On the contrary, methods of making nanopores by applying energy to cell membranes, for example, technologies such as electroporation or microneedle, have the advantage of being able to deliver relatively various materials to various cells. However, low cell viability due to the invasiveness of the methods, denaturation of a delivery material, and low throughput have been pointed out as major limitations. In order to solve such problems, the use of microfluidic devices capable of processing a large amount of cells is remarkable. Representatively, there is a platform that creates a narrow constriction or bottleneck in the microchannels and creates nanopores in the cell membrane through physical deformation of the cells when the cells pass through the narrow constriction or bottleneck. However, this approach has major disadvantages such as clogging of the narrow constriction or bottleneck itself, inconsistent material delivery efficiency, and the like during the experiment.

For example, US Patent No. 2014-0287509 (hereinafter, the conventional technology) discloses a technique of inducing cell transformation by directly flowing cells through a channel having a bottleneck structure, thereby applying pressure to the cells. However, in this case, since the cells progress at a non-uniform speed, the rate at which the cells are transformed is not constant, and thus the efficiency of material delivery is reduced. In addition, since cells are delivered only by a method due to diffusion, the efficiency of material delivery is fundamentally low and there is a problem in that it is difficult to deliver nucleic acids into the nucleus. Therefore, it is urgent to develop an innovative next-generation intracellular material delivery platform that can deliver various materials uniformly and with high efficiency into the cells while maintaining the high processing function of the microfluidic device.

## DISCLOSURE OF THE INVENTION

### TECHNICAL PROBLEM

An object of the present disclosure is to provide a platform capable of delivering materials into cells by generating vortices in microchannels.

### TECHNICAL SOLUTION

According to one aspect of the present disclosure, one embodiment of the present disclosure may include: a first channel through which a fluid containing cells and a delivery material flows while having a flow; and a second channel and a third channel which are connected to the first channel at an angle so that the fluid containing the cells and the delivery material flows while having a flow; wherein the fluid forms at least one of a collision region and a vortex region of the fluid in at least one of the first channel, the second channel, and the third channel.

In one embodiment, the cross section of at least one of the first to third channels may be formed as a rectangle having a short axis and a long axis, the short axis is provided as a vertical plane, and the long axis is provided as a horizontal plane.

In one embodiment, the material delivery platform may further include: a first supply part supplying a first supply fluid containing at least one of cells and a delivery material; and a second supply part supplying a second supply fluid, wherein the first supply fluid may be supplied to the first channel through a first supply part channel, the second supply fluid may be supplied to the first channel through second supply part channels, one or more of the second supply part channels may be formed, and the second supply part channels may supply the second fluid from both sides of the first supply part channel.

In one embodiment, the Reynolds number of the first supply part channel to the Reynolds number of the second supply part channel may be 2:1 to 1:3.

In one embodiment, the fluid in the first channel may include the first supply fluid and the second supply fluid, the first supply fluid may flow by being flowed from the center of the long axis in the first channel, the second supply fluid may flow by being flowed from both sides of the first supply fluid and flow by being flowed from both ends of the long axis in the first channel, and the first supply fluid and the second supply fluid may be mixed in at least one of the second channel and the third channel.

In one embodiment, the vortex region may be one in which a straight line-type flow of the fluid is temporarily stagnant.

In one embodiment, the delivery material may be at least one of nucleic acids, proteins, fluorescent dyes, quantum dots, carbon nanotubes, antigens, ribonucleoproteins, genetic scissors, polymers, and nanoparticles.

In one embodiment, the second channel and the third channel may be formed symmetrically or asymmetrically with respect to the first channel.

In one embodiment, the angle between the second and third channels and the first channel may be at least one of an acute angle, a right angle, and an obtuse angle.

In one embodiment, the cross section of at least one of the first channel, the second channel, and the third channel may have a rectangular shape having a long axis and a short axis, the long axis may be provided as a horizontal plane, the short axis may be provided as a vertical plane, the long axis may be 10 um to 10 mm, and the short axis may be 5 um to 60 $\mu$m.

In one embodiment, the fluid containing the cells and the delivery material may have a Reynolds number of 1 to 1,000 according to Equation 1 below in the first channel, and at least one of the second channel and the third channel may have a Reynolds number of 1 to 1,000 according to Equation 1 below.

[Equation 1]

$$Re = \frac{\rho VD}{\mu}$$

(In Equation 1, $\mu$ is a viscous coefficient of the fluid, $\rho$ is a density, V is an average velocity of the fluid, and D is a hydraulic diameter of the tube.)

In one embodiment, the Reynolds number of the second channel or the third channel may be provided at 40% to 110% of the Reynolds number of the first channel.

In one embodiment, the at least one of the first to third channels may have a hydraulic diameter of 5 um to 130 um according to Equation 2 below.

[Equation 2]

$$D = \frac{4A_c}{P}$$

(In Equation 2, $A_c$ is a cross-sectional area of the tube through which the fluid flows, and P is a length of the two-dimensional curve that surrounds and is in contact with the fluid when looking at the cross section.)

In one embodiment, the at least one of the cells and the delivery material of the first channel may have a particle Reynolds number of 4 to 100 defined by Equation 3.

[Equation 3]

$$Re_P = Re\left(\frac{a}{D}\right)^2$$

(In Equation 3, $Re_p$ is a particle Reynolds number, Re is a Reynolds number, a is a diameter of the cell or particle, and D is a hydraulic diameter.)

In one embodiment, the hydraulic diameter of the second channel or the third channel may be provided at 40% to 110% of the hydraulic diameter of the first channel.

In one embodiment, the at least one of a first vortex and a second vortex may be formed in the vortex region, the first vortex may be formed in a portion where the first channel and the second channel, and the third channel may be connected to each other, and the second vortex may be formed in at least one of the second channel and the third channel.

In one embodiment, the second vortex may be formed in at least one of the second channel and the third channel, and the second vortex may be formed in a portion spaced apart 20 um to 200 um in the longitudinal direction of the second or third channel from the central portion of the first channel.

In one embodiment, the at least one of the first vortex and the second vortex may be formed by a change in pressure of the fluid.

In one embodiment, the first vortex or the second vortex may be formed stronger as the Reynolds number of the fluid increases.

In one embodiment, the time during which the flow of the cells is stagnant by the second vortex may be 0.1 us to 100 us.

In one embodiment, the at least one of the collision, the first vortex, and the second vortex may form a temporary perforation in at least one of a cell membrane and a nuclear membrane of the cells, and the delivery material may flow into the cells through the perforation.

In one embodiment, the perforation may be formed in at least one of the cell membrane and the nuclear membrane of the cells by at least one of the collision, the first vortex, and the second vortex, the perforation may be formed in the same site or a different site of the cell membrane or the nuclear membrane, a next perforation may be formed after a perforation formed as a first one is maintained or restored, and the perforation formed as a first one may be extended larger or maintained in size by the next perforation.

In one embodiment, the second channel and the third channel may be connected to each other at the end of the first channel to branch the fluid flowing in the first channel, a protruding groove may be formed between the second channel and the third channel, and the protruding groove may be formed by being protruded in a direction in which the fluid flows at a position corresponding to the first channel.

In one embodiment, the protruding groove may have a vertical cross section formed in at least one of a quadrangular shape, a triangular shape, and a cylindrical shape.

In one embodiment, the introduction part of the protruding groove may be 1 um to 20 um, and the depth of the protruding groove may be 3 um to 100 $\mu$m.

In one embodiment, the at least one of a first vortex and a second vortex may be formed in the vortex region, the first vortex may be formed by local pressure reversal after the fluid passing through the first channel is collided with at least one of the introduction part of the protruding groove, the inside of the protruding groove, and the partition wall around the protruding groove, and the second vortex may be formed by local pressure reversal in each of the second channel and the third channel.

## ADVANTAGEOUS EFFECTS

According to the present disclosure as described above, it is possible to provide a platform for delivering a delivery material into cells by generating multiple vortices. In addition, the delivery material may include various delivery materials such as nucleic acids, proteins, and nanoparticles, and a combination of two or more such as nucleic acids and proteins, in which only one of these is not specified, can provide a platform for intracellular delivery.

## BRIEF DESCRIPTION OF THE DRAWINGS

FIG. 1 is views schematically showing that cells and delivery materials flow in a platform and the delivery materials are delivered into the cells according to one embodiment of the present disclosure.

FIG. 2 is views schematically showing that cells, a delivery material, a fluid, etc. are delivered from a first supply part or a second supply part to a first channel according to one embodiment of the present disclosure.

FIG. 3 schematically shows a form in which a first supply fluid or a fluid flows by flowing from a first supply part channel or a first channel.

FIG. 4 is views simulating a process of forming a first vortex according to one embodiment of the present disclosure.

FIG. 5 is a view showing an appearance in which a first vortex is formed in a Y-shaped channel according to one embodiment of the present disclosure.

FIG. 6 is a view showing an appearance in which a second vortex is formed in the Y-shaped channel according to one embodiment of the present disclosure.

FIG. 7 is graphs showing efficiencies of delivering the delivery materials into cells in the Y-shaped channel according to one embodiment of the present disclosure.

FIG. 8 is a view showing an appearance in which cells are deformed according to the formation of a first vortex in an arrow ($\rightarrow$) shaped channel according to one embodiment of the present disclosure.

FIG. 9 is a view showing an appearance in which cells are deformed according to the formation of a second vortex in an arrow-shaped channel according to one embodiment of the present disclosure.

FIG. 10 is graphs showing efficiencies of delivering the delivery materials into cells in the arrow-shaped channel according to one embodiment of the present disclosure.

FIG. 11 is graphs showing efficiencies and viabilities of delivery materials delivered into cells using the platform according to one embodiment of the present disclosure.

## BEST MODE FOR CARRYING OUT THE INVENTION

According to one aspect of the present disclosure, one embodiment of the present disclosure may include: a first channel through which a fluid containing cells and a delivery material flows while having a flow; and a second channel and a third channel which are connected to the first channel at an angle so that the fluid containing the cells and the delivery material flows while having a flow; wherein the fluid forms at least one of a collision region and a vortex region of the fluid in at least one of the first channel, the second channel, and the third channel.

## MODE FOR CARRYING OUT THE INVENTION

Details of other embodiments are included in the detailed description and drawings.

Advantages and features of the present disclosure, and methods of achieving them, will become clear with reference to the detailed description of the following embodiments taken in conjunction with the accompanying drawings. However, the present disclosure is not limited to the embodiments disclosed below, but may be implemented in various different forms, and since, unless otherwise specified in the following description, these numbers are essentially approximations that reflect the various uncertainties of measurement that arise in obtaining these values among other things, it should be understood that all numbers, values and/or expressions expressing components, reaction conditions, or amounts of the components in the present disclosure are in all instances modified by the term "about". Also, when a numerical range is disclosed in the present description, such a range is continuous and includes all values in such a range from a minimum value to the maximum value including a maximum value, unless otherwise indicated. Furthermore, when such a range refers to an integer, all integers including from the minimum value to the maximum value including a maximum value are included unless otherwise indicated.

Further, when a range is stated for a variable in the present disclosure, it will be understood that the variable includes all values within the stated range including the stated endpoints of the range. For example, a range of "5 to 10" includes any subrange of 6 to 10, 7 to 10, 6 to 9, 7 to 9, and the like as well as values of 5, 6, 7, 8, 9, and 10, and it will be understood also to include any value between integers that fall within the scope of the stated range, such as 5.5, 6.5, 7.5, 5.5 to 8.5, 6.5 to 9, and the like. For example, a range of "10% to 30%" includes any subrange of 10% to 15%, 12% to 18%, 20% to 30%, and the like as well as values such as 10%, 11%, 12%, 13% and the like, and all integers including up to 30%, and it will be understood also to include any value between integers that fall within the scope of the stated range, such as 10.5%, 15.5%, 25.5%, and the like.

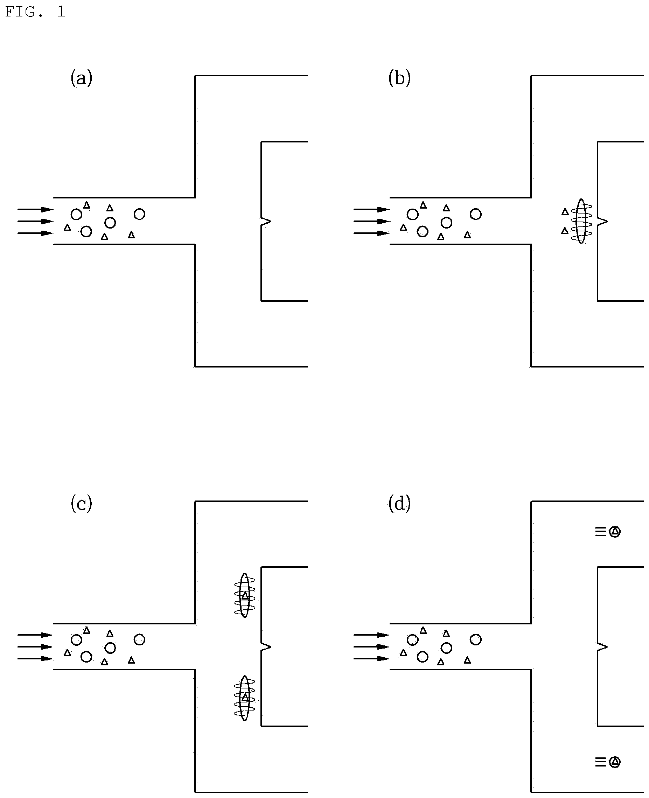

FIG. 1 is views schematically showing an appearance in which vortexes are formed and fluids flow in intracellular material delivery platforms according to one embodiment of the present disclosure. FIG. 1 illustrates one embodiment of the present disclosure, but is not limited thereto. The platforms of FIG. 1 are a T-shaped platform, but the same process may be performed on a Y-shaped or arrow-shaped (→) platform.

According to one aspect of the present disclosure, one embodiment of the present disclosure may include: a first channel through which a fluid containing cells and a delivery material flows while having a flow; and a second channel and a third channel which are connected to the first channel at an angle so that the fluid containing the cells and the delivery material flows while having a flow; wherein the fluid forms at least one of a collision region and a vortex region of the fluid in at least one of the first channel, the second channel, and the third channel.

The first channel may be a channel into which a fluid including cells and a delivery material is flowed while having a flow. The fact that the fluid has a flow in the first channel may include flowing it at a constant speed due to an external force being applied along with simply diffusing the fluid. Specifically, the flow of the fluid in the first channel may be controlled by an external force, and thus the cells and the delivery material flowing together with the fluid in the first channel may also flow at a constant speed.

The fluid may represent all fluids flowing through the first to third channels. As the fluid, the first supply fluid and the second supply fluid flow separately in the first channel, but the first supply fluid and the second supply fluid may flow in a mixture in the second or third channel.

A cross section of at least one of the first to third channels may be formed as a rectangle having a short axis and a long axis. The long axis may be provided parallel to the floor with respect to the bottom surface, that is, provided as a horizontal plane. The short axis may be provided almost vertically with respect to the bottom surface, that is, provided as a vertical plane. However, this is only an example, and the shapes of the first to third channels may not be limited thereto.

The material delivery platform may further include: a first supply part supplying a first supply fluid containing at least one of cells and a delivery material; and a second supply part supplying a second supply fluid, wherein the first supply fluid may be supplied to the first channel through a first supply part channel, the second supply fluid may be supplied to the first channel through second supply part channels, one or more of the second supply part channels may be formed, and the second supply part channels may supply the second fluid from both sides of the first supply part channel.

The first supply part may supply the first supply fluid to the first channel. The first supply fluid may be a hydrophilic fluid containing another material. The another material may be at least one of a cell, a delivery material, and a medium.

The first supply part may apply an external force to the first supply fluid to flow the first supply fluid toward the first channel through the first supply part channel. Accordingly, the first supply fluid may flow at a constant speed in the first supply part channel or the first channel.

The second supply part may supply the second supply fluid to the first channel. The second supply fluid may be a hydrophilic fluid, for example, water. The second supply fluid may include a delivery material.

The second supply part may apply an external force to the second supply fluid to flow the second supply fluid toward the first channel through the second supply part channel. Accordingly, the second supply fluid may flow at a constant speed in the second supply part channel or the first channel.

In the first supply part channel or the second supply part channel, the first supply fluid or the second supply fluid may flow while having a certain Reynolds number. The Reynolds number of the first supply part channel and the Reynolds number of the second supply part channel may be 2:1 to 1:3. Preferably, the Reynolds number of the first supply part channel and the Reynolds number of the second supply part channel may be 1:1. When there are two or more second supply part channels, the respective Reynolds numbers of the second supply part channels may be the same.

In the second supply part channel, a plurality of channels in one second supply part may be connected to the first channel. Alternatively, in the second supply part channel, the respective channels in a plurality of second supply parts may be connected to the first channel.

For example, when there is one of the second supply part and there are two second supply part channels connected thereto, the Reynolds number of the second supply part may be twice or more than the Reynolds number of the first supply part. In this case, the Reynolds numbers of the first supply part and the second supply part may be 1:1 to 1:3.

Also, for example, when there are two of the second supply parts and there is one second supply part channel connected to each thereof, the Reynolds number of the second supply part may be the same as or slightly higher than that of the first supply part. In this case, the Reynolds numbers of the first supply part and the second supply part may be 1:1 to 1:1.5.

There may be two or more of the second supply part channels, and the second supply part channels may be formed on both sides with the first supply part channel interposed therebetween. Accordingly, the second supply fluid may be supplied to both sides with the first supply fluid interposed therebetween and supplied to the first channel.

Specifically, in the first channel, the first supply fluid may flow by flowing at the center of the long axis in the first channel, and the second supply fluid may flow by flowing at both sides of the first supply fluid, that is, both ends of the long axis in the first channel. It can flow by floating at the end. For example, when the long axis of the first channel is divided into thirds, the second supply fluid may flow up to a first 1/3 point, the first supply fluid may flow up to a second 1/2 point, and the second supply fluid may flow by being flowed up to a last 1/3 point again.

Both the first supply fluid and the second supply fluid may include a hydrophilic fluid, but may not be mixed in the first channel. The first supply fluid and the second supply fluid may flow while having a constant speed and a constant Reynolds number, and as a result, the fluids and materials contained in each of the fluids may not be mixed or diffused. In addition, the fact that the first supply fluid and the second supply fluid are not mixed may be influenced by the size and shape of the first channel.

That is, the first supply fluid may be guided to flow in the center of the first channel by the second supply fluid. The first supply fluid may pass through the first channel to collide with a protruding groove formed facing the center of the first channel or near the protruding groove, and thus cells contained in the first supply fluid may collide with the protruding groove or near the protruding groove.

The second channel and the third channel may be channels connected to ends of the first channel.

The second channel and the third channel may be connected to the first channel at a predetermined angle to receive a fluid of the first channel. The angle between the second and third channels and the first channel may be at least one of an acute angle, a right angle, and an obtuse angle. Accordingly, the second channel and the third channel may be formed in an arrow (→) shape, a T shape, or a Y shape based on the first channel. Preferably, the second channel and the third channel may be formed in an arrow (→) shape or a T shape based on the first channel.

The second channel and the third channel may be formed symmetrically or asymmetrically with respect to the first channel. Preferably, the second channel and the third channel may be formed symmetrically with respect to the first channel. In other words, the second channel and the third channel may be formed symmetrically with respect to the longitudinal direction of the first channel.

A cross section of at least one of the first channel, the second channel, and the third channel may have a rectangular shape having a long axis and a short axis, the long axis may be provided as a horizontal plane, the short axis may be provided as a vertical plane, the long axis may be 10 um to 10 mm, and the short axis may be 5 um to 60 um. The length of the short axis or the long axis may be in a range in which the first supply fluid and the second supply fluid can flow without mixing in the first channel. In addition, the length of the short axis or the long axis may be in a range in which vortices can be well formed in the second channel and the third channel.

The first to third channels may be provided at a length ratio of a long axis and a short axis of 2:1, and may be provided in a rectangular shape of, for example, $80 \times 40 \mu m$ or $40 \times 30 \mu m$.

The vortex region may be a portion where a straight line-type flow of the first fluid is temporarily stagnant.

The delivery material may be applied without limitation as long as it is a material that can be delivered into cells. The delivery material may be, for example, at least one of nucleic acids, proteins, fluorescent dyes, quantum dots, carbon nanotubes, antigens, ribonucleoproteins, genetic scissors, polymers, and nanoparticles.

The fluid including the cells and the delivery material may have a Reynolds number of 1 to 1,000 according to Equation 1 below in the first to third channels. If the Reynolds number is less than 1, vortexes may not be formed since a sufficient amount and speed are not formed to generate vortices in the channels, and if the Reynolds number is greater than 1,000, an unstable fluid flow may be generated.

[Equation 1]

$$Re = \frac{\rho VD}{\mu}$$

In Equation 1, $\mu$ is a viscous coefficient of the fluid, $\rho$ is a density, V is an average velocity of the fluid, and D is a hydraulic diameter of the tube.

The Reynolds number of the second channel or the third channel may be 40% to 110% with respect to the Reynolds number of the first channel.

In addition, the second channel or the third channel may be provided to have a Reynolds number smaller than that of the first channel. For example, the second channel or the third channel may be provided to have a Reynolds number that is 50% to 80% of the Reynolds number of the first channel.

In addition, at least one of the first to third channels may have a hydraulic diameter of 1 um to 100 mm according to Equation 2 below. Also, the hydraulic diameter according to the Equation 2 may be preferably 5 um to 130 um. The hydraulic diameter can define the size of each channel. Therefore, when each channel is not a cylinder, the size of each channel can be determined by the hydraulic diameter. The hydraulic diameter in Equation 2 may be the hydraulic diameter in Equation 1 above.

[Equation 2]

$$ D = \frac{4A_c}{P} $$

In Equation 2, $A_c$ is a cross-sectional area of the tube through which the fluid flows, and P is a length of the two-dimensional curve that surrounds and is in contact with the fluid when looking at the cross section.

When the channel has a hydraulic diameter of smaller than 5 $\mu$m, the amount of fluid flowing in the channel may not be sufficient to form a vortex. In addition, when the channel has a hydraulic diameter of larger than 130 $\mu$m, a force (for example, pressure reversal) capable of forming a vortex may not be formed in the flow of the fluid.

The second channel or the third channel may be provided to have a hydraulic diameter that is 40% to 110% based on the hydraulic diameter of the first channel.

In addition, the second channel or the third channel may be provided to have a hydraulic diameter of smaller than that of the first channel. For example, the second channel or the third channel may be provided to have a hydraulic diameter of that is 50% to 80% of the hydraulic diameter of the first channel.

In addition, the fluid of the first channel may be defined by a particle Reynolds number ($Re_p$) of Equation 3.

[Equation 3]

$$ Re_P = Re \left( \frac{a}{D} \right)^2 $$

(In Equation 3, $Re_p$ is a particle Reynolds number, Re is a Reynolds number, a is a diameter of the cell or particle, and D is a hydraulic diameter.)

For example, when the particle Reynolds number is about 1, the first supply fluid delivered to the first channel may flow by flowing in the center of the first channel. Accordingly, in this case, the cells may flow to the center of the first channel even without the second supply part channel.

In addition, for example, when the particle Reynolds number is between 4 and 100, the second supply fluid guides the first supply fluid so that the first supply fluid may flow by flowing in the center of the first channel.

Accordingly, the particle Reynolds number in the first channel may be 0.5 to 100. In addition, the particle Reynolds number in the first channel may be 1 to 100. In addition, the particle Reynolds number in the first channel may be preferably 4 to 100. In addition, the particle Reynolds number in the first channel may be preferably 25 to 40.

At least one of portions where the first to third channels and the first channel and the second and third channels meet may include a vortex region. The straight line-type flow of the fluid flowing through the first to third channels may be temporarily stagnant in the vortex region. The vortex region may be a portion where a vortex is formed in the channel.

A protruding groove may be formed in a portion where the first channel is connected to the second and third channels. The protruding groove may be formed by protruding in a direction in which a fluid flows at a position corresponding to the first channel.

The protruding groove may diverge a fluid flowing in the first channel by connecting the second channel and the third channel to each other at an end of the first channel.

The protruding groove may have a vertical cross section formed of at least one of a quadrangular shape, a triangular shape, and a cylindrical shape. The protruding groove may be in the form of a tube with a closed end, and may not be limited to the above example as a form capable of generating a vortex.

In addition, the introduction part of the protruding groove may be 1 um to 20 um, and the depth of the protruding groove may be 3 um to 100 $\mu$m.

The cells coming out of the first channel may collide with the introduction part of the protruding groove or may be flowed into the protruding groove and collide with each other. Even when the cells flow into the protruding groove and collide with each other, they may escape out of the protruding groove after the collision and undergo a vortex.

Alternatively, the cells may collide with a partition wall around the protruding groove.

At least one of a first vortex and a second vortex may be formed in the vortex region. Specifically, the first vortex may be formed in a portion where the first channel and the second channel are connected and a portion where the first channel and the third channel are connected to each other. Also, specifically, the second vortex may be formed in at least one of the second channel and the third channel.

The vortex may refer to a flow in which the fluid encounters a sudden change in pressure, an obstacle, etc. to interrupt the flow so that the proceeding direction is changed. That is, the vortex may be formed while the fluid flowing through the channel collides with an obstacle (e.g., a protruding groove or a partition wall) or a straight line flow is stagnant due to reversal of pressure, etc. Accordingly, when a vortex is formed in the fluid, cells and/or a delivery material contained in the fluid may receive an external force while undergoing the vortex, and in this process, deformation may occur in the cells.

In more detail, the first vortex may be formed in a stagnation point region. The stagnation point region may be included in a portion where the first channel is connected to the second and third channels. The stagnation point region may be a portion where the fluid flowing out of the first channel is stagnant to form the first vortex. That is, the stagnation point region may be a portion where the fluid flowing out of the first channel collides with at least one of the protruding groove and the partition wall to form a first vortex. Accordingly, the stagnation point region may be a portion where the cells and/or the delivery material contained in the fluid collide with at least one of the protruding groove and the partition wall and undergo a first vortex. However, the cells may undergo only one of the collision and the first vortex, and even in this case, deformation may occur in the cells.

"Collision" of the cells may be a meaning including all that the cells collide with at least one of the introduction part of the protruding groove, the inside of the protruding groove, and the partition wall. Here, the partition wall may be a partition wall around the protruding groove.

In addition, the second vortex may be formed in at least one of the second channel and the third channel. A site where the second vortex is formed may be a site completely out of the end of the first channel. In other words, the site where the second vortex is formed may be a site completely out of a region extending in the same width as the width of the first channel in the longitudinal direction of the first channel. Specifically, the site where the second vortex is formed may be formed in a portion spaced apart from the central portion of the first channel (or the central portion of the protruding groove) by 20 um to 200 um in the longitudinal direction of the second or third channel.

At a point ahead of 20 um in the longitudinal direction of the second channel (or the third channel), the first fluid flowing in the first channel may interfere with the formation of a vortex so that the vortex may not be formed well. In addition, at a point behind than 200 um in the longitudinal direction of the second channel (or the third channel), the fluid may not have a sufficient velocity or Reynolds number to form the vortex.

The second vortex may be formed by a change in pressure of the fluid in the second channel or the third channel, and the flow of the fluid may be stagnant at a portion where the second vortex is formed. Therefore, the flow of the cells and the delivery material contained in the fluid may be stagnant.

The fact that the flow of the fluid, cells, and delivery material is stagnant (or stopped) may mean that the straight line flow is stagnant, and may not mean a state without movement. When the flow of the fluid, cells and delivery material is stagnant, they can move in a vortex shape riding on the flow of the vortex. That is, the fluid, cells, and delivery material may be stagnant by the vortex.

The time at which the cells are stopped (stagnant) by the second vortex may be 0.1 $\mu$s to 100 $\mu$s.

The first vortex or the second vortex may be formed stronger as the Reynolds number of the fluid increases. Accordingly, when the Reynolds number of the first channel is greater than that of the second channel and/or the third channel, the first vortex may be formed to be stronger than the second vortex. In this case, the cells may be deformed more by the first vortex.

The fluid flowing through the first to third channels and the cells and delivery material contained in the fluid may undergo at least one of the collision, the first vortex, and the second vortex. Therefore, even when the cells undergo only one of the collision, the first vortex, and the second vortex, the cells may be deformed and the delivery material may be delivered into the cells.

When cells are deformed by the collision or vortex, a temporary perforation may be formed in at least one of a cell membrane and a nuclear membrane, and the delivery material may flow into the cells through the perforation.

At this time, the perforation may be formed in the same site or different site of the cell membrane or the nuclear membrane, and a next perforation may be formed after a perforation formed as a first one is maintained or restored. The perforation formed as a first one may be extended larger by the next perforation.

The cells may undergo all of the collision, the first vortex, and the second vortex, and at this time, the delivery material may be better delivered into the cells.

When the delivery material is delivered into the cells by the collision or vortex, the delivery material may be delivered into the cells when deformation occurs only in the cells. However, when deformation occurs in the cell membrane and the nuclear membrane, the delivery material may be delivered to at least one of the inside of the cells and the inside of the nucleus.

The delivery material may include all materials having a size smaller than that of the cells. The delivery material may be, for example, at least one of nucleic acids, proteins, and nanoparticles. Specifically, the delivery material may be each composed of nucleic acids, proteins, and nanoparticles, and may include various combinations of one or more, such as a combination of nucleic acids, proteins, and nanoparticles, depending on the use.

At least one of a first vortex and a second vortex may be formed in the vortex region, the first vortex may be generated after the fluid passing through the first channel collides with the protruding groove or the partition wall, and the second vortex may be formed by a local increase in pressure in each of the second and third channels.

Specifically, the cells may receive a two-step force in the stagnation point region. As a first step, the cells collide with the introduction part of the protruding groove or the inside of the protruding groove, or collide with the partition wall to receive a force, and thus deformation may occur in the cells. As a second step, the cells leave the protruding groove or the partition wall, and the flow of the fluid leaving the protruding groove or the partition wall is mixed with the flow of the first channel to form a first vortex, and thus deformation may occur in the cells. However, the cells may undergo only one of the first and second steps.

Since the cells undergo the first vortex after collision, they may be deformed to a greater extent in the stagnation point region. In addition, the force by which the first vortex deforms the cells may be greater than the force by the second vortex.

Meanwhile, the second vortex may be generated when a pressure increases in a local portion of the second channel and/or the third channel. This is because the fluid flows from a high pressure side to a low pressure side in the second channel and/or the third channel, but there may be a portion where the pressure is reversed in a local portion of the second channel (or third channel), where the second vortex may be formed.

The first vortex and the second vortex may be sequentially formed based on one of the cells or delivery material. However, the first vortex and the second vortex may be formed simultaneously or at different times based on the platform according to one embodiment of the present disclosure.

Hereinafter, examples and comparative examples of the present disclosure will be described. However, the following examples are only preferred embodiments of the present disclosure, and the scope of rights of the present disclosure is not limited by the following examples.

FIG. 1 is views schematically showing that a fluid containing cells and a delivery material undergoes a first vortex and a second vortex in a T-shaped channel according to one embodiment of the present disclosure.

FIG. 1A shows that a fluid containing cells and a delivery material flows at a constant speed in a first channel. In FIG. 1A, circles represent cells and triangles represent delivery materials, and these float and flow in a fluid.

FIG. 1B shows an appearance in which the cells undergo a first vortex by colliding with the protruding groove or the partition wall and then leaving the protruding groove or the partition wall. Although not shown in this drawing, the cells may be deformed to generate a perforation while colliding with the protruding groove or the partition wall, and thus the delivery material may be delivered. In addition, even while the cells undergo the first vortex, the cells may be deformed to generate a perforation, and the delivery material may be delivered into the cells. While the cells undergo at least one of the collision and the first vortex, a perforation may be generated in at least one of the cell membrane and the nuclear membrane, and the delivery material may be delivered into the cells (or into the nucleus) through the perforation.

FIG. 1C shows an appearance in which cells and delivery materials flowed into the second channel or the third channel undergo a second vortex in a middle portion of the channel. Here, the cells undergoing the second vortex may have already undergone at least one of collision with the protruding groove, collision with the partition wall, or the first vortex. Also, the cells undergoing the second vortex may be cells that have not undergone either the collision or the first vortex. Here, the delivery material may be delivered again into the cells where the delivery material is already present. In addition, the delivery material may also be delivered into the cells where the delivery material is not present.

FIG. 1D shows an appearance in which the cells escape the channel after receiving the delivery material. These cells may be obtained and used in various fields.

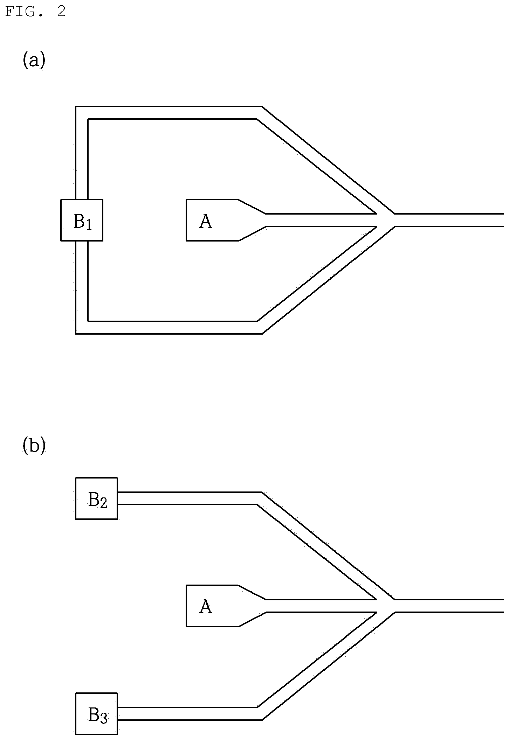

FIG. 2 schematically shows a system in which cells, a delivery material, a fluid, etc. are delivered to a first channel in a first supply part and a second supply part. A represents a first supply part, and at least one of the cells, the delivery material, and the medium flows in the first supply fluid and is supplied to the first channel. B1 to B3 represent a second supply part, and the delivery material or the like flows in the second supply fluid and is supplied to the first channel.

A plurality of second supply part channels in one second supply part may be connected to the first channel (upper FIG. 2), or each of the second supply part channels in the plurality of second supply parts may be connected to the first channel (lower FIG. 2).

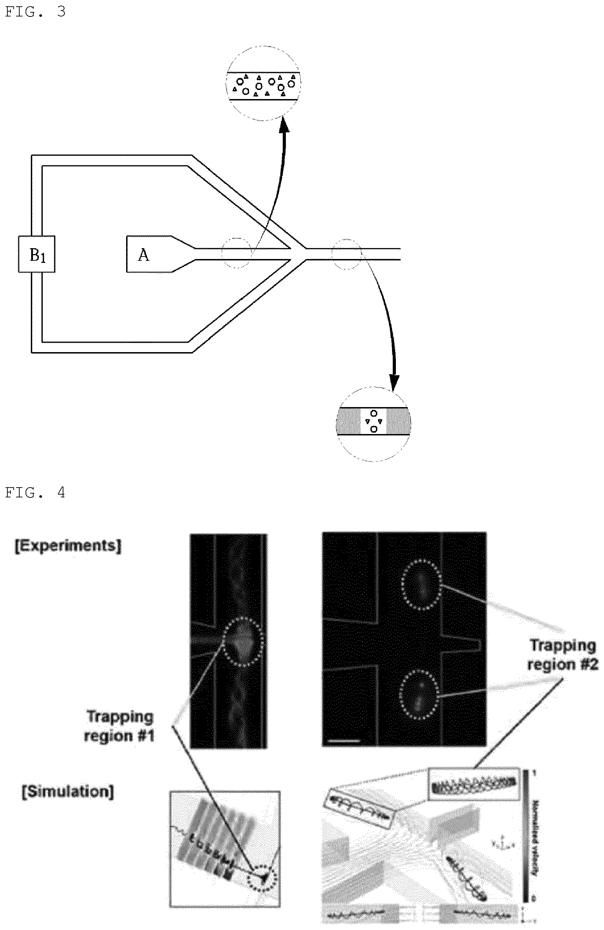

FIG. 3 shows a cross section of the channel in the upper drawing of FIG. 2, and schematically shows a form in which the first supply fluid or the fluid flows in the first supply part channel or the first channel. In the first supply part channel, a fluid containing cells or delivery materials flows throughout the channel. However, the second supply fluid supplied from the second supply part channel further exists in the first channel, and the first supply fluid and the second supply fluid flow without mixing. As shown in the drawing, the first supply fluid delivered from the first supply part channel flows to the center of the first channel, and the cells contained therein flow to the center of the first supply fluid.

Both of the first supply fluid and the second supply fluid may include fluids of the same nature, but the fluids are not mixed or the material exchange is not performed due to the velocity or channel shape of these fluids.

FIG. 4 is results of visualizing through micro-fluorescent particles a process in which the fluid flowing out of the first channel collides with the protruding groove or the partition wall to form the first vortex, and simulating the visualized process. It can be confirmed that the cells and micro-fluorescent particles collide with the partition wall through the movement path and then move while being affected by the vortex.

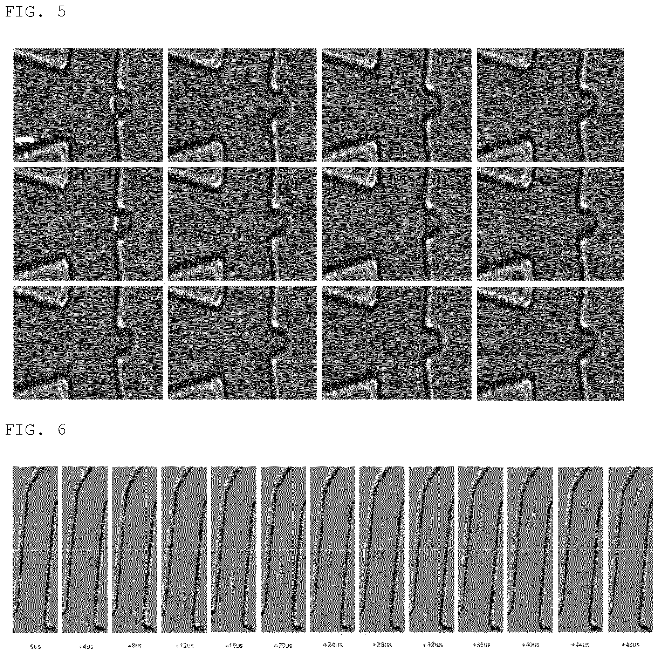

FIGS. 5 to 7 show the process and efficiency of material delivery into the cells by forming a vortex in a Y-shaped channel, which is one embodiment of the present disclosure. Here, the platform used a first channel with a cross-sectional diameter of 80 × 40 um, and the Reynolds number in the first channel of the fluid was 300.

FIG. 5 shows an appearance in which cells contained in the fluid flowing out of the first channel collide with the protruding groove so that the shape of the cells is deformed, and the cells undergo a first vortex. It can be confirmed that the cells colliding with the protruding groove are deformed in the form depending on the shape of the protruding groove, or the form is deformed by the first vortex.

In addition, FIG. 6 shows an appearance in which the fluid undergoes a second vortex while flowing through the second channel (or the third channel). It can be confirmed that the flow of the cells in the section of 24 $\mu$s to 32 $\mu$s is stagnant at about the midpoint of the channel. Such a stagnation occurs when the second vortex is formed in the channel, and in this process, the cells are deformed, and thus the material is delivered into the cells.

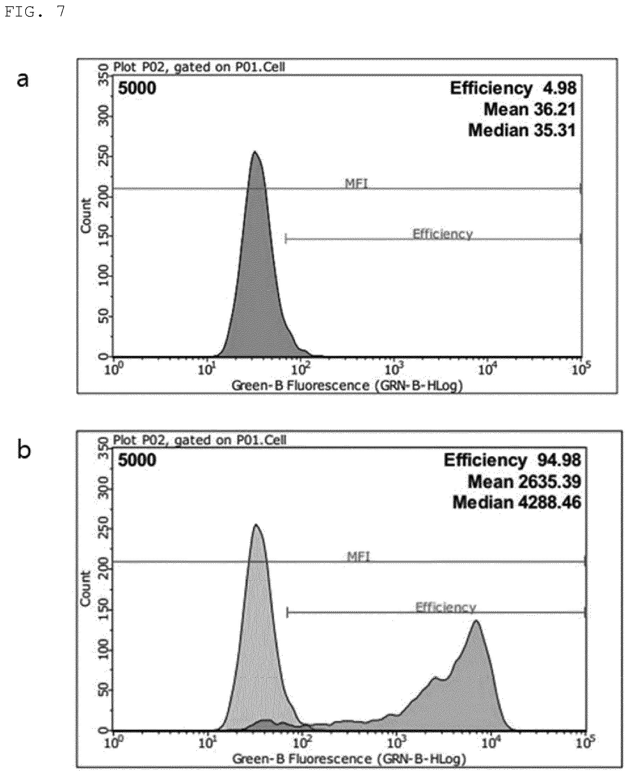

FIG. 7 is results of delivering a fluorescent material to each cell and quantitatively measuring the t phase of fluorescence (FITC-Dextran) using flow cytometry. A total of 5000 cells were measured, and the x-axis represents fluorescence intensity, and the y-axis represents the count of cells. Using this, it is possible to check the delivery efficiency of the delivery material into the cells on this platform.

FIG. 7A is a view of measuring cells that were not subjected to the platform treatment as a control group. Therefore, since a fluorescent material is hardly contained, and thus it is located on the left side of the graph. Meanwhile, FIG. 7B shows the control group (right) and the experimental group (left) overlapped together. In the experimental group, a lot of the fluorescent material was delivered to the cells, showing a right-skewed appearance.

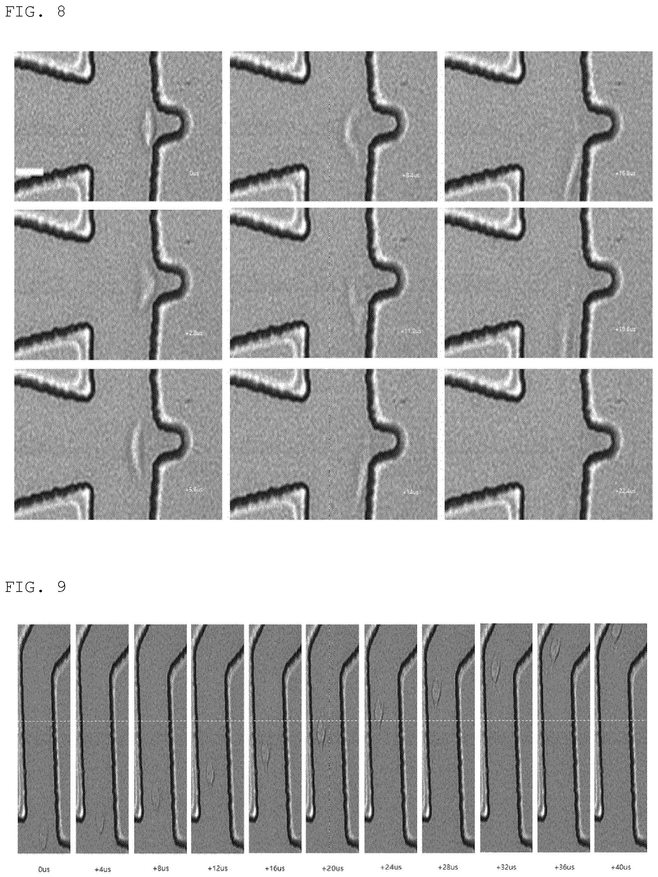

FIGS. 8 to 10 show the process and efficiency of a material being delivered into cells by forming a vortex in an arrow-shaped channel, which is one embodiment of the present disclosure. Here, the platform used a first channel with a cross-sectional diameter of 80 × 40 um, and the Reynolds number in the first channel of the fluid was 300.

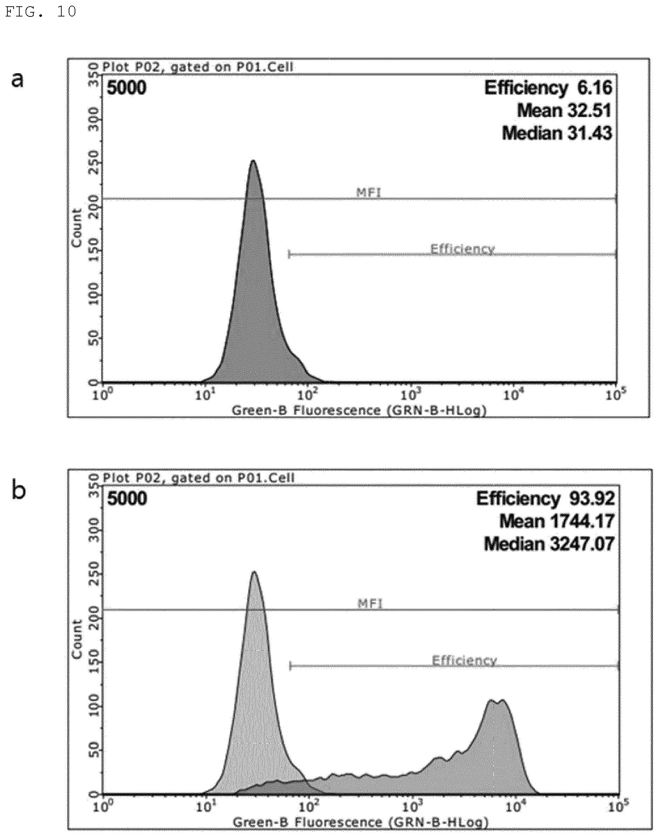

In FIGS. 8 and 10, it can be confirmed that the shape of the cells is deformed while the cells undergo the first vortex or the second vortex similarly to the Y-shaped channel. In FIG. 9, it can be confirmed that the flow of the cells in the section of 20 $\mu$s to 24 $\mu$s is stagnant at about the midpoint of the channel by the second vortex.

FIG. 10 is results of delivering a fluorescent material to each cell and quantitatively measuring the t phase of fluorescence (FITC-Dextran) using flow cytometry under the same conditions as in FIG. 7. In FIG. 10B, it can be confirmed that the cells passing through the platform express fluorescence, and thus the graph is skewed to the right.

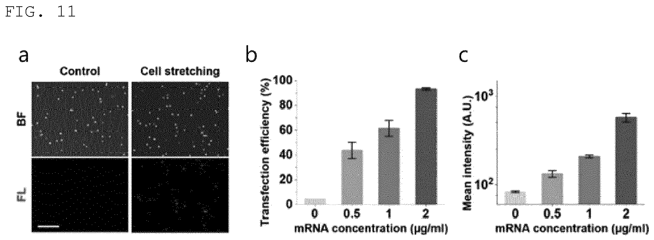

FIG. 11 is graphs confirming efficiencies and viabilities of delivery materials delivered into cells using the platform according to one embodiment of the present disclosure.

mRNA expressing green fluorescent protein (GFP) was delivered to K562 cells. In FIG. 11A, fluorescence was not expressed in the control group, but it can be confirmed that fluorescence was expressed in the experimental group (cell stretching).

FIG. 11B is a graph showing delivery efficiencies according to mRNA concentrations. The higher the mRNA concentration, the higher the delivery efficiency, and the efficiency was about 90% even at a very low mRNA concentration of 2 $\mu$g/ml.

In addition, the results in FIG. 11B are graphed again as ratios of absolute fluorescence intensities in FIG. 11C. The average fluorescence intensity when the mRNA concentration was 2 ug/ml was shown to be about 10 times stronger than when mRNA was not present.

Those skilled in the art to which the present disclosure pertains will understand that the present disclosure can be embodied in other specific forms without changing its technical spirit or essential features. Therefore, the embodiments described above should be understood as illustrative in all respects and not limiting. The scope of the present disclosure is indicated by the claims to be described later rather than the detailed description above, and all changes or modified forms derived from the meaning and scope of the claims and equivalent concepts thereof should be interpreted to be included in the scope of the present disclosure.

**Claims**

1. An intracellular material delivery platform, comprising:

a first channel through which a fluid containing cells and a delivery material flows while having a flow; and

a second channel and a third channel which are connected to the first channel at an angle so that the fluid containing the cells and the delivery material flows while having a flow;

wherein the fluid forms at least one of a collision region and a vortex region of the fluid in at least one of the first channel, the second channel, and the third channel.

2. The intracellular material delivery platform of claim 1, wherein the cross section of at least one of the first to third channels is formed as a rectangle having a short axis and a long axis, the short axis is provided as a vertical plane, and the long axis is provided as a horizontal plane.

3. The intracellular material delivery platform of claim 1, wherein the material delivery platform further comprise: a first supply part supplying a first supply fluid containing at least one of cells and a delivery material; and a second supply part supplying a second supply fluid, wherein the first supply fluid is supplied to the first channel through a first supply part channel, the second supply fluid is supplied to the first channel through second supply part channels, one or more of the second supply part channels are formed, and the second supply part channels supply the second fluid from both sides of the first supply part channel.

4. The intracellular material delivery platform of claim 3, wherein the Reynolds number of the first supply part channel to the Reynolds number of the second supply part channel is 2:1 to 1:3.

5. The intracellular material delivery platform of claim 3, wherein the fluid in the first channel includes the first supply fluid and the second supply fluid, the first supply fluid flows by being flowed from the center of the long axis in the first channel,

the second supply fluid flows by flowing from both sides of the long axis in the first channel with the second supply fluid being at both sides of the first supply fluid, and the first supply fluid and the second supply fluid are mixed in at least one of the second channel and the third channel.

6. The intracellular material delivery platform of claim 1, wherein the vortex region is one in which a straight line-type flow of the fluid is temporarily stagnant.

7. The intracellular material delivery platform of claim 1, wherein the delivery material is at least one of nucleic acids, proteins, fluorescent dyes, quantum dots, carbon nanotubes, antigens, ribonucleoproteins, genetic scissors, polymers, and nanoparticles.

8. The intracellular material delivery platform of claim 1, wherein the second channel and the third channel are formed symmetrically or asymmetrically with respect to the first channel.

9. The intracellular material delivery platform of claim 1, wherein the angle between the second and third channels and the first channel is at least one of an acute angle, a right angle, and an obtuse angle.

10. The intracellular material delivery platform of claim 1, wherein the cross section of at least one of the first channel, the second channel, and the third channel has a rectangular shape having a long axis and a short axis, the long axis is provided as a horizontal plane, the short axis is provided as a vertical plane, the long axis is 10 um to 10 mm, and the short axis is 5 um to 60 $\mu$m.

11. The intracellular material delivery platform of claim 1, wherein the fluid containing the cells and the delivery material has a Reynolds number of 1 to 1,000 according to Equation 1 below in the first channel, and at least one of the second channel and the third channel has a Reynolds number of 1 to 1,000 according to Equation 1 below.

[Equation 1]

$$Re = \frac{\rho VD}{\mu}$$

(In Equation 1, $\mu$ is a viscous coefficient of the fluid, $\rho$ is a density, $V$ is an average velocity of the fluid, and $D$ is a hydraulic diameter of the tube.)

12. The intracellular material delivery platform of claim 11, wherein the Reynolds number of the second channel or the third channel is provided at 40% to 110% of the Reynolds number of the first channel.

13. The intracellular material delivery platform of claim 11, wherein the at least one of the first to third channels has a hydraulic diameter of 5 um to 130 um according to Equation 2 below.

[Equation 2]

$$D = \frac{4A_c}{P}$$

(In Equation 2, $A_c$ is a cross-sectional area of the tube through which the fluid flows, and P is a length of the two-dimensional curve that surrounds and is in contact with the fluid when looking at the cross section.)

14. The intracellular material delivery platform of claim 1, wherein the at least one of the cells and the delivery material of the first channel has a particle Reynolds number of 0.5 to 100 defined by Equation 3 below.

[Equation 3]

$$Re_P = Re\left(\frac{a}{D}\right)^2$$

(In Equation 3, $Re_p$ is a particle Reynolds number, Re is a Reynolds number, a is a diameter of the cell or particle, and D is a hydraulic diameter.)

15. The intracellular material delivery platform of claim 10, wherein the hydraulic diameter of the second channel or the third channel is provided at 40% to 110% of the hydraulic diameter of the first channel.

16. The intracellular material delivery platform of claim 1, wherein the at least one of a first vortex and a second vortex is formed in the vortex region, the first vortex is formed in a portion where the first channel and the second channel, and the third channel are connected to each other, and the second vortex is formed in at least one of the second channel and the third channel.

17. The intracellular material delivery platform of claim 16, wherein the second vortex is formed in at least one of the second channel and the third channel, and the second vortex is formed in a portion spaced apart 20 um to 200 um in the longitudinal direction of the second or third channel from the central portion of the first channel.

18. The intracellular material delivery platform of claim 16, wherein the at least one of the first vortex and the second vortex is formed by a change in pressure of the fluid.

19. The intracellular material delivery platform of claim 16, wherein the first vortex or the second vortex is formed stronger as the Reynolds number of the fluid increases.

20. The intracellular material delivery platform of claim 16, wherein the time during which the flow of the cells is stagnant by the second vortex is 0.1 $\mu$s to 100 $\mu$s.

21. The intracellular material delivery platform of claim 16, wherein the at least one of the collision, the first vortex, and the second vortex forms a temporary perforation in at least one of a cell membrane and a nuclear membrane of the cells, and the delivery material flows into the cells through the perforation.

22. The intracellular material delivery platform of claim 16, wherein the perforation is formed in at least one of the cell membrane and the nuclear membrane of the cells by at least one of the collision, the first vortex, and the second vortex, the perforation is formed in the same site or a different site of the cell membrane or the nuclear membrane, a next perforation is formed after a perforation formed as a first one is maintained or restored, and the perforation formed as a first one is extended larger or maintained in size by the next perforation.

23. The intracellular material delivery platform of claim 1, wherein the second channel and the third channel are connected to each other at the end of the first channel to branch the fluid flowing in the first channel, a protruding groove is formed between the second channel and the third channel, and the protruding groove is formed by being protruded in a direction in which the fluid flows at a position corresponding to the first channel.

24. The intracellular material delivery platform of claim 23, wherein the protruding groove has a vertical cross section formed in at least one of a quadrangular shape, a triangular shape, and a cylindrical shape.

25. The intracellular material delivery platform of claim 23, wherein the introduction part of the protruding groove is 1 um to 20 um, and the depth of the protruding groove is 3 um to 100 $\mu$m.

26. The intracellular material delivery platform of claim 23, wherein the at least one of a first vortex and a second vortex is formed in the vortex region, the first vortex is formed by local pressure reversal after the fluid passing through the first channel is collided with at least one of the introduction part of the protruding groove, the inside of the protruding groove, and the partition wall around the protruding groove, and the second vortex is formed by local pressure reversal

**EP 4 324 926 A1**

in each of the second channel and the third channel.

FIG. 1

FIG. 2

(a)

(b)

FIG. 3

FIG. 4

FIG. 5

FIG. 6

FIG. 7

FIG. 8

FIG. 9

0us +4us +8us +12us +16us +20us +24us +28us +32us +36us +40us

FIG. 10

FIG. 11

## INTERNATIONAL SEARCH REPORT

| International application No. |
|---|
| **PCT/KR2022/005272** |

### A. CLASSIFICATION OF SUBJECT MATTER

**C12N 15/87**(2006.01)i; **C12M 1/42**(2006.01)i; **C12M 3/06**(2006.01)i

According to International Patent Classification (IPC) or to both national classification and IPC

### B. FIELDS SEARCHED

Minimum documentation searched (classification system followed by classification symbols)

C12N 15/87(2006.01); B01L 3/00(2006.01); C12N 15/89(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세포 내 전달(intracellular delivery), 미세유체 기기(microfluidic device)

### C. DOCUMENTS CONSIDERED TO BE RELEVANT

| Category* | Citation of document, with indication, where appropriate, of the relevant passages | Relevant to claim No. |
|---|---|---|
| Y | KR 10-2020-0109228 A (KOREA UNIVERSITY RESEARCH AND BUSINESS FOUNDATION) 22 September 2020 (2020-09-22)<br>See abstract; claims 1-3 and 6; paragraphs [0021], [0030] and [0037]-[0038]; and figures 2 and 4-5. | 1-26 |
| Y | KANG, G. Y. et al. Intracellular Nanomaterial Delivery via Spiral Hydroporation. ACS Nano. 2020, vol. 14, pp. 3048-3058.<br>See page 3050; and figure 1. | 1-26 |
| Y | CAPRETTO, L. et al. Production of polymeric micelles by microfluidic technology for combined drug delivery: Application to osteogenic differentiation of human periodontal ligament mesenchymal stem cells (hPDLSCs). International Journal of Pharmaceutics. vol. 440, 2013, pp. 195-206.<br>See abstract; page 199; and figure 1. | 3-5 |
| A | JARRELL, J. A. et al. Intracellular delivery of mRNA to human primary T cells with microfuidic vortex shedding. Scientific Reports. 2019, vol. 9, thesis no.:3214, pp. 1-11.<br>See entire document. | 1-26 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **15 July 2022** | **18 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/005272** |

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2014-0116374 A (MASSACHUSETTS INSTITUTE OF TECHNOLOGY) 02 October 2014 (2014-10-02)<br>  See entire document. | 1-26 |

Form PCT/ISA/210 (second sheet) (July 2019)

# EP 4 324 926 A1

<table>
<tr><td colspan="4" align="center"><b>INTERNATIONAL SEARCH REPORT</b><br><b>Information on patent family members</b></td><td colspan="2">International application No.<br><b>PCT/KR2022/005272</b></td></tr>
<tr><td colspan="2" align="center">Patent document<br>cited in search report</td><td align="center">Publication date<br>(day/month/year)</td><td colspan="2" align="center">Patent family member(s)</td><td align="center">Publication date<br>(day/month/year)</td></tr>
<tr><td colspan="2">KR    10-2020-0109228    A</td><td>22 September 2020</td><td>AU</td><td>2020-234406    A1</td><td>21 October 2021</td></tr>
<tr><td colspan="2"></td><td></td><td>CA</td><td>3133045    A1</td><td>17 September 2020</td></tr>
<tr><td colspan="2"></td><td></td><td>CN</td><td>113840657    A</td><td>24 December 2021</td></tr>
<tr><td colspan="2"></td><td></td><td>EP</td><td>3939701    A1</td><td>19 January 2022</td></tr>
<tr><td colspan="2"></td><td></td><td>JP</td><td>2022-528826    A</td><td>16 June 2022</td></tr>
<tr><td colspan="2"></td><td></td><td>KR</td><td>10-2020-0109229    A</td><td>22 September 2020</td></tr>
<tr><td colspan="2"></td><td></td><td>KR</td><td>10-2217407    B1</td><td>22 February 2021</td></tr>
<tr><td colspan="2"></td><td></td><td>KR</td><td>10-2354673    B1</td><td>25 January 2022</td></tr>
<tr><td colspan="2"></td><td></td><td>US</td><td>2022-0177819    A1</td><td>09 June 2022</td></tr>
<tr><td colspan="2"></td><td></td><td>WO</td><td>2020-184992    A1</td><td>17 September 2020</td></tr>
<tr><td colspan="2">KR    10-2014-0116374    A</td><td>02 October 2014</td><td>AU</td><td>2012-326203    A1</td><td>15 May 2014</td></tr>
<tr><td colspan="2"></td><td></td><td>AU</td><td>2012-326203    A2</td><td>22 May 2014</td></tr>
<tr><td colspan="2"></td><td></td><td>AU</td><td>2012-326203    B2</td><td>30 November 2017</td></tr>
<tr><td colspan="2"></td><td></td><td>BR</td><td>112014009346    A2</td><td>18 April 2017</td></tr>
<tr><td colspan="2"></td><td></td><td>BR</td><td>112014009346    B1</td><td>15 September 2020</td></tr>
<tr><td colspan="2"></td><td></td><td>CA</td><td>2852672    A1</td><td>25 April 2013</td></tr>
<tr><td colspan="2"></td><td></td><td>CA</td><td>2852672    C</td><td>20 July 2021</td></tr>
<tr><td colspan="2"></td><td></td><td>CN</td><td>103987836    A</td><td>13 August 2014</td></tr>
<tr><td colspan="2"></td><td></td><td>CN</td><td>103987836    B</td><td>10 April 2018</td></tr>
<tr><td colspan="2"></td><td></td><td>CN</td><td>107058101    A</td><td>18 August 2017</td></tr>
<tr><td colspan="2"></td><td></td><td>CN</td><td>107058101    B</td><td>01 June 2021</td></tr>
<tr><td colspan="2"></td><td></td><td>CN</td><td>113337402    A</td><td>03 September 2021</td></tr>
<tr><td colspan="2"></td><td></td><td>DK</td><td>2768942    T3</td><td>27 January 2020</td></tr>
<tr><td colspan="2"></td><td></td><td>EP</td><td>2768942    A1</td><td>27 August 2014</td></tr>
<tr><td colspan="2"></td><td></td><td>EP</td><td>2768942    B1</td><td>04 December 2019</td></tr>
<tr><td colspan="2"></td><td></td><td>EP</td><td>3608394    A1</td><td>12 February 2020</td></tr>
<tr><td colspan="2"></td><td></td><td>ES</td><td>2764105    T3</td><td>02 June 2020</td></tr>
<tr><td colspan="2"></td><td></td><td>HR</td><td>P20200051    T1</td><td>20 March 2020</td></tr>
<tr><td colspan="2"></td><td></td><td>HU</td><td>E047507    T2</td><td>28 April 2020</td></tr>
<tr><td colspan="2"></td><td></td><td>JP</td><td>2014-533936    A</td><td>18 December 2014</td></tr>
<tr><td colspan="2"></td><td></td><td>JP</td><td>2018-023395    A</td><td>15 February 2018</td></tr>
<tr><td colspan="2"></td><td></td><td>JP</td><td>6219832    B2</td><td>25 October 2017</td></tr>
<tr><td colspan="2"></td><td></td><td>JP</td><td>6629272    B2</td><td>15 January 2020</td></tr>
<tr><td colspan="2"></td><td></td><td>KR</td><td>10-2058568    B1</td><td>22 January 2020</td></tr>
<tr><td colspan="2"></td><td></td><td>LT</td><td>2768942    T</td><td>10 April 2020</td></tr>
<tr><td colspan="2"></td><td></td><td>PL</td><td>2768942    T3</td><td>18 May 2020</td></tr>
<tr><td colspan="2"></td><td></td><td>PT</td><td>2768942    T</td><td>21 January 2020</td></tr>
<tr><td colspan="2"></td><td></td><td>RS</td><td>59898    B1</td><td>31 March 2020</td></tr>
<tr><td colspan="2"></td><td></td><td>RU</td><td>2014119926    A</td><td>27 November 2015</td></tr>
<tr><td colspan="2"></td><td></td><td>RU</td><td>2656156    C2</td><td>31 May 2018</td></tr>
<tr><td colspan="2"></td><td></td><td>SI</td><td>2768942    T1</td><td>31 March 2020</td></tr>
<tr><td colspan="2"></td><td></td><td>US</td><td>10696944    B2</td><td>30 June 2020</td></tr>
<tr><td colspan="2"></td><td></td><td>US</td><td>2014-0287509    A1</td><td>25 September 2014</td></tr>
<tr><td colspan="2"></td><td></td><td>US</td><td>2019-0093073    A1</td><td>28 March 2019</td></tr>
<tr><td colspan="2"></td><td></td><td>US</td><td>2020-0277566    A1</td><td>03 September 2020</td></tr>
<tr><td colspan="2"></td><td></td><td>WO</td><td>2013-059343    A1</td><td>25 April 2013</td></tr>
</table>

Form PCT/ISA/210 (patent family annex) (July 2019)

**EP 4 324 926 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140287509 A